# EUROPEAN PATENT SPECIFICATION

(11) **EP 2 691 028 B1**
(45) Date of publication and mention of the grant of the patent: **01.06.2016**
(21) Application number: 12715781.6
(22) Date of filing: 02.04.2012
(51) Int. Cl.: A61B 17/04, A61B 17/06

(54) **MEDICAL SUTURING IMPLEMENT**
MEDIZINISCHE NAHTVORRICHTUNG
INSTRUMENT MÉDICAL À SUTURER

(30) Priority: 31.03.2011 JP 2011077756
(43) Date of publication of application: 05.02.2014
(73) Proprietor: Covidien LP, Mansfield, MA 02048 (US)
(72) Inventor: SAKAI, Yosuke, Fukuroi-shi Shizuoka 437-0004 (JP)
(74) Representative: Tomlinson, Edward James
(86) International application number: PCT/US2012/031790
(87) International publication number: WO 2012/135806

(56) References cited:
- WO-A1-2009/113221
- US-A- 5 722 981
- US-A1- 2005 043 746
- US-A1- 2009 264 905

## Description

### FIELD OF THE INVENTION

The present invention relates to a medical suturing implement used when introducing suture thread inside a visceral organ and pulling it out from inside the visceral organ, wherein the suture thread holds the visceral organ wall of an organ such as the stomach or bladder on the body surface side of the abdominal wall, etc., from outside the body.

### BACKGROUND

In the past, in people with reduced ability to ingest food orally by themselves due to old age or illness (called "patients" hereinafter), parenteral nutrition has been performed, whereby liquid foods, nutrients, etc., are supplied using a fistula catheter. For example, in cases where percutaneous endoscopic gastrostomy (PEG) is used, a through-hole (for example, a fistula such as a gastric fistula) which penetrates the abdominal wall and the visceral organ wall (gastric wall) of the patient is constructed, a fistula catheter is mounted in this through-hole, and liquid foods, etc., are supplied to the patient via the fistula catheter.

To easily form the through-hole, normally, the visceral organ wall, which tends to readily move, and the abdominal wall are percutaneously affixed by suturing using suture thread when constructing the through-hole. Thus, a variety of medical suturing implements used to affix the visceral organ wall and abdominal wall by suturing have been proposed.

As an example of such implements, a medical device which, "inside a case, houses a feeding mechanism which sequentially feeds suture thread that was inserted inside from the base end of a suture thread insertion puncture needle 20 toward the tip end, and a protrusion mechanism which causes a ring-shaped component of a stylet housed inside a suture thread grasping puncture needle 30 to protrude from the tip end of the suture thread grasping puncture needle 30" has been disclosed (for example, refer to Japanese Unexamined Patent Application Publication No. 2009-213763 and Japanese Unexamined Patent Application Publication No. 2009-213764). This medical device enables ligation inside the body to be efficiently and safely performed by only one technician or by a technician and an assistant.

WO 2009/113221 discloses a medical suturing tool comprising the features of the preamble of claim 1.

In the medical device described in the prior art, the technician must insert the suture thread inside the medical device, which increases the amount of labor for the technician. Also, the manipulations related to inserting suture thread is often intricate, and manipulability is poor. Additionally, in the prior art, after the suture thread insertion puncture needle and the suture thread grasping puncture needle are stuck into the stomach from the abdominal wall, an operation to push in the stylet to form the ring-shaped component into a ring, an operation to turn an operating roller to insert the suture thread into the stomach, and an operation to press a release button to grasp the suture thread with the ring-shaped component are required, and operability is poor.
The present invention was brought about to solve the problems described above, and provides a medical suturing implement which greatly improves manipulability and operability.
The medical suturing implement according to the present invention is defined in independent claim 1. Preferred embodiments are defined in the dependent claims.

By the medical suturing implement according to the present invention, simply by push-pull operation of the operating part, suture thread can be introduced inside a visceral organ and pulled it out from inside the visceral organ, and as a result, the burden of operation on the technician can be greatly reduced, and operability is markedly improved.

### BRIEF DESCRIPTION OF THE DRAWINGS

FIG. 1 is a schematic exterior view illustrating an example of the configuration of the medical suturing implement according to embodiment 1 of the present invention.
FIG. 2 is a schematic perspective view illustrating the exterior configuration of the medical suturing implement according to embodiment 1 of the present invention.
FIG. 3 is a schematic interior configuration drawing illustrating an example of the interior configuration of the medical suturing implement according to embodiment 1 of the present invention.
FIG. 4 is a schematic cross-sectional view which illustrates the operating procedure of the medical suturing implement according to embodiment 1 of the present invention in a simplified manner.
FIG. 5 is a schematic cross-sectional view which illustrates the operating procedure of the medical suturing implement according to embodiment 1 of the present invention in a simplified manner.
FIG. 6 is a schematic cross-sectional view which illustrates the operating procedure of the medical suturing implement according to embodiment 1 of the present invention in a simplified manner.
FIG. 7 is an explanatory diagram for explaining the operation of the medical suturing implement according to embodiment 1 of the present invention.
FIG. 8 is a schematic interior configuration view illustrating an example of the interior configuration of the medical suturing implement according to embodiment 2 of the present invention.
FIG. 9 is a schematic view illustrating an another example of the configuration of the suture thread feeding mechanism.

### DETAILED DESCRIPTION

Embodiments of the present invention will be described below based on the drawings.

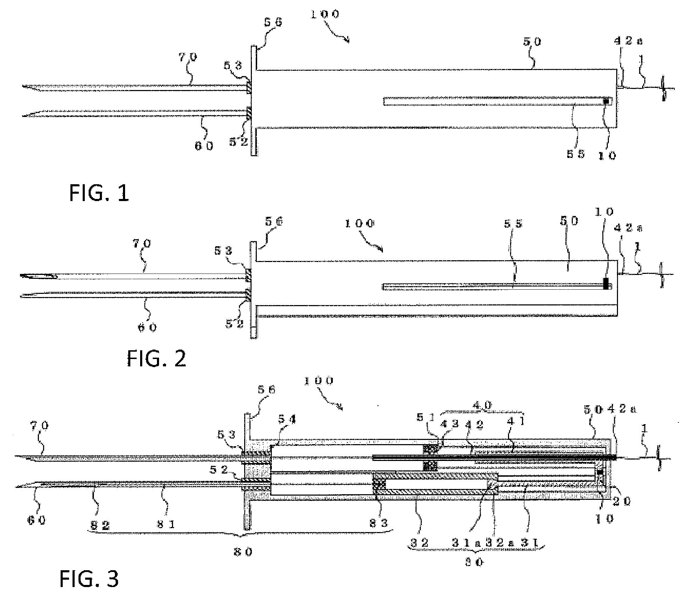

FIG. 1 is a schematic exterior view illustrating an example of the configuration of the medical suturing implement according to embodiment 1 of the present invention (called simply "suturing implement 100" hereinafter). FIG. 2 is a perspective view illustrating the exterior configuration of the suturing implement 100. FIG. 3 is a schematic interior configuration drawing illustrating an example of the interior configuration of the suturing implement 100. The suturing implement 100 will now be explained based on FIG. 1-FIG. 3. To make it easy to form a fistula into which a fistula catheter is inserted, the suturing implement 100 is used when introducing suture thread inside a visceral organ, wherein the suture thread lifts and holds the visceral organ wall of an organ such as the stomach or bladder on the body surface side of the abdominal wall from outside the body. Note that there are cases where the relationships among the sizes of the constituent components differ in the drawings below, including FIG. 1.

The suturing implement 100 comprises a housing 50, an operating part 10 by which a technician performs operations, a coupling part 20 which transfers the movement of the operating part 10 to a loop feeding/returning mechanism 30 and a suture thread feeding mechanism 40, a loop feeding/returning mechanism 30 which operates coupled with the movement of the operating part 10 via the coupling part 20, a suture thread feeding mechanism 40 which operates coupled with the movement of the operating part 10 via the coupling part 20, a loop introduction needle 60 provided on the tip side of the housing 50, a suture thread introduction needle 70 in which suture thread is housed, provided on the tip side of the housing 50 substantially parallel to and separated by a prescribed distance from the loop introduction needle 60, and a loop forming part 80 which is moveably inserted inside the loop introduction needle 60.

Note that in FIG. 1-FIG. 3, the suture thread 1 which is fed out from the tip end (blade edge) of the suture thread introduction needle 70 by the suture thread feeding mechanism 40 is also illustrated. Further, the "tip side" is the name for the side inserted into the patient (patient side), and the "base side" is the name for the side operated by the technician (operating side). In the descriptions below, there are also cases where the tip side is called the "forward direction" and the base side is called the "backward direction." Additionally, the suture thread 1 has a length for suturing multiple times.

### Suture Thread 1

The suture thread 1 is not a constituent of the suturing implement 100, but since it functions as an organ fixing implement, it is described briefly here. The suture thread 1 may be constructed of a material that has enough flexibility to yield to body tissue when passed through inside the body, and has enough tensile strength to lift an organ (for example, nylon thread, etc.). Also, the suture thread 1 is cut when attached to or removed from the patient. For this reason, the suture thread 1 is preferably constructed of a material and with a diametric dimension such that it can be cut with a tool found at a medical treatment site such as scissors. Also, the suturing implement 100 is stuck into the patient in the state where the suture thread 1 has been inserted up to the tip end of the suture thread introduction needle 70.

### Housing 50

The housing 50 is a hollow form, constructed in a substantially rectangular shape such that the forward/backward directions are its long direction. The housing 50 houses the loop feeding/returning mechanism 30 and the suture thread feeding mechanism 40. The loop feeding/returning mechanism 30 and the suture thread feeding mechanism 40 are housed so as to be aligned substantially in parallel in the housing 50, and they can move in the forward/backward directions inside the housing 50. The housing 50 unifies the entire suturing implement 100, and also serves as the part that is actually held when it is operated by the technician. Note that the housing 50 does not have to have a substantially rectangular shape, and its exterior shape is not particularly limited. In short, the housing 50 can be configured in any way, provided that it can allow the other configurations described below to function.

Further, on the tip side of the housing 50, the loop introduction needle 60 and suture thread introduction needle 70 are attached substantially in parallel and separated by a prescribed distance. For example, as shown in FIG. 1, supporting parts (supporting part 52, supporting part 53) may be provided on the tip side of the housing 50, and the base ends of the loop introduction needle 60 and suture thread introduction needle 70 may be inserted into the supporting parts, and the loop introduction needle 60 and suture thread introduction needle 70 may be detachably supported. The supporting parts may be a material such as synthetic resin molded into a rectangular shape, for example. Note that the base ends of the loop introduction needle 60 and suture thread introduction needle 70 may be supported in advance by the supporting components, and the supporting components may be detachably attached on the tip side of the housing 50. Also, the loop introduction needle 60 and suture thread introduction needle 70 may also be affixed to the housing 50 in advance. Additionally, the loop introduction needle 60 and suture thread introduction needle 70 do not have to be mounted at the very tip end of the housing 50, and can be attached on the tip side of the housing 50. In short, the base sides of the loop introduction needle 60 and suture thread introduction needle 70 may extend inside the housing 50.

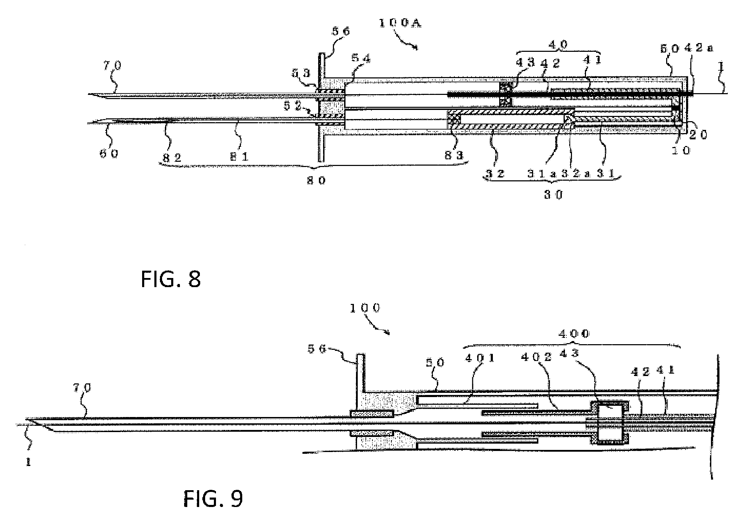

The tip surface of a hollow component for loop introduction 32 which constitutes the loop feeding/returning mechanism 30 and the tip surface of a suture thread fastening component 43 which constitutes the suture thread feeding mechanism 40 contact the tip side inner wall surface of the housing 50 (called simply "inner wall surface 54" hereinafter) - in short, they run into it. When the tip end of the hollow component for loop introduction 32 which constitutes the loop feeding/returning mechanism 30 and the tip end of the suture thread fastening component 43 which constitutes the suture thread feeding mechanism 40 run into the inner wall surface 54 of the housing 50, their forward-direction movement beyond that is restricted. Also, on a part of the inner wall surface on the side surface side of the housing 50, a stopper 51 is formed, which restricts movement of the suture thread fastening component 43 in the backward direction. As the stopper 51, a part of the inner wall surface of the housing 50 may protrude toward the inside of the housing 50, or a step may be provided on a part of the inner wall surface of the housing 50. However, in the case where a guide tube has been provided, as shown in FIG. 9, for example, on the tip side of the housing 50 - that is, on the tip side on the inside of the housing 50 - the tip end of the hollow component for loop introduction 32 and the suture thread fastening component 43 may contact the guide tube in a prescribed location.

Also, in the side surface of the housing 50, a guide hole 55 through which the operating part 10 can move in the forward/backward directions is formed along the long direction of the housing 50. This guide hole 55 is formed so as to penetrate the side surface of the housing 50. Note that the constituent material of the housing 50 is not particularly limited, and may be a synthetic resin such as vinyl chloride, polycarbonate, or a polyolefin such as polypropylene or polyethylene. Note that on the tip end outer wall of the housing 50, a flange part 56 is provided, which protrudes like a flange to the outside in the radial direction. The flange part 56 is there to assist operation.

### Operating Part 10

The operating part 10 is provided inside the housing 50 or in the guide hole 55 of the housing 50 so as to protrude from the side surface of the housing 50. It is operated by the technician when introducing an organ fixing implement inside a visceral organ using the suturing implement 100. The operating part 10 can move in the forward/backward directions along the guide hole 55 formed in a part of the side surface of the housing 50. Note that the constituent material of the operating part 10 is not particularly limited, and may be the same synthetic resin as the housing 50, or it can be a constituent material different from that of the housing 50.

FIG. 1-FIG. 3 illustrate an example of the operating part 10 having a shape such that it protrudes outward from the side surface of the housing 50 in a direction orthogonal to the direction of advancement. By the operating part 10 having such a shape, the technician can easily apply his finger to it, and operability is improved. However, the shape of the operating part 10 is not limited to the shape shown in FIG. 1 and FIG. 2. For example, it may also be configured such that the base end is extended backward, and a rod which protrudes from the base side of the housing 50 is provided, and this rod, serving as the operating part 10, can be pushed and pulled in and out of the inside of the housing 50. Operability may also be improved by providing a button, etc., on the base end of the rod. Also, in cases where the operating part 10 is provided inside the housing 50, the operating part 10 can be operated by inserting the finger via the guide hole 55.

### Coupling Part 20

The coupling part 20 operates coupled with the movement of the operating part 10. It transfers the movement of the operating part 10 to the loop feeding/returning mechanism 30 and suture thread feeding mechanism 40 housed in the housing 50. As shown in FIG. 3, in the coupling part 20, one side (the side not on the operating part 10 side) is divided into two branches, where one of the two branches is coupled to the loop feeding/returning mechanism 30 and the other of the two branches is coupled to the suture thread feeding mechanism 40. The other side (operating part 10 side) is coupled to the operating part 10, and as a result, it can transfer the movement of the operating part 10 to the loop feeding/returning mechanism 30 and suture thread feeding mechanism 40.

FIG. 3 shows an example of the state where the loop introduction plunger 31 which constitutes the loop feeding/returning mechanism 30 and the suture thread introduction plunger 41 which constitutes the suture thread feeding mechanism 40 are integrated, and the coupling part 20 is configured as a slider which moves in the backward/forward directions. In short, the coupling part 20 moves the loop introduction plunger 31 and suture thread introduction plunger 41 by sliding in accordance with the movement of the operating part 10.

Note that the shape of the coupling part 20 is not particularly limited, and may be any shape provided that it can transfer the movement of the operating part 10 to the loop feeding/returning mechanism 30 and suture thread feeding mechanism 40. Also, the coupling part 20 may be formed integrated with the operating part 10, or it may be formed as a separate body from the operating part 10 that can couple with the operating part 10. Additionally, the coupling part 20 may be formed integrated with the loop introduction plunger 31 and suture thread introduction plunger 41, or it may be formed as a body separate from the loop introduction plunger 31 and the suture thread introduction plunger 41 that can couple with the loop introduction plunger 31 and suture thread introduction plunger 41. Further, a guide for moving the coupling part 20 straight back and forth (for example, a groove into which the coupling part 20 fits) may be formed inside the housing 50.

### Loop Feeding/Returning Mechanism 30

The loop feeding/returning mechanism 30 couples the loop forming part 80, which has been moveably inserted inside the loop introduction needle 60, to the movement of the operating part 10, and feeds it to the tip side of the loop introduction needle 60 and returns it to inside the housing 50. The loop feeding/returning mechanism 30 is constructed from the loop introduction plunger 31 and the hollow component for loop introduction 32 which joins with the loop forming part 80.

The loop introduction plunger 31 is coupled to the coupling part 20, and can move inside the housing 50 in the forward/backward directions in accordance with the movement of the operating part 10. In the loop introduction plunger 31, the tip end is inserted inside the base side of the hollow component for loop introduction 32 such that they can couple, and by the coupled state being released, it can move in the forward/backward directions inside the hollow component for loop introduction 32. In the descriptions below, the coupling part of the tip side of the loop introduction plunger 31 is called the tip side coupling part 31a, and the coupling part of the base side of the hollow component for loop introduction 32 is called the base side coupling part 32a. The coupled state of the tip side coupling part 31a and base side coupling part 32a is released by the operating part 10 moving further forward in the state where the hollow component for loop introduction 32 has run into the inner wall surface 54 of the housing 50.

Thus, when the loop introduction plunger 31 is moved in the forward/backward directions in the state where it is coupled with the hollow component for loop introduction 32, the hollow component for loop introduction 32 also moves in the forward/backward directions. On the other hand, when the loop introduction plunger 31 is moved in the forward/backward directions in the state where it is not coupled with the hollow component for loop introduction 32, the loop introduction plunger 31 moves in the forward/backward directions inside the hollow component for loop introduction 32 independent of the hollow component for loop introduction 32. FIG. 3 shows an example of the state where the loop introduction plunger 31 can move in the forward/backward directions inside the hollow component for loop introduction 32, but the base side of the hollow component for loop introduction 32 may be inserted in the tip side of the loop introduction plunger 31, and the hollow component for loop introduction 32 may be able to move in the backward/forward directions inside the loop introduction plunger 31. Note that a guide for moving the loop introduction plunger 31 straight back and forth (for example, a groove into which the loop introduction plunger 31 fits) may be formed inside the housing 50.

The tip side coupling part 31a and the base side coupling part 32a may be configured in a shape such that they fit together by tapering (refer to FIGS. 3-6). Specifically, they may be configured such that the tip side coupling part 31a has a tapered shape that gradually widens toward the tip end, and the base side coupling part 32a has a tapered shape that gradually widens toward the tip end, and the tip side coupling part 31a and the base side coupling part 32a are coupled by utilizing the frictional force that arises between the tapered outer wall surface of the tip side coupling part 31a and the tapered inner wall surface of the base side coupling part 32a. Therefore, if the tip side coupling part 31a and the base side coupling part 32a are configured in a shape such that they can fit together by tapering, the insertion friction can be adjusted by the tapering angles of the two, and the coupled state between the two can be firmly maintained with a simple structure.

However, the tip side coupling part 31a and the base side coupling part 32a are not limited to a shape such that they can be coupled by fitting together by tapering. For example, a protuberance which protrudes outward in the radial direction may be provided on the tip side coupling part 31 a or the base side coupling part 32a, and a fitting part that fits on that protuberance may be provided on the tip side coupling part 31a or the base side coupling part 32a, so as to couple them. In this case, the protuberance and the fitting part may be fit together or released by either the loop introduction plunger 31 or the hollow component for loop introduction 32 being rotated. Also, a guide groove may also be formed so as to guide the protuberance to the fitting part. In addition, it may be configured such that the protuberance is removed from the fitting part when at least a prescribed force is applied.

The hollow component for loop introduction 32 is formed in a hollow shape, and the loop introduction plunger 31 is inserted such that it can move in the forward/backward directions. Also, the loop forming part 80 is connected to the tip side of the hollow component for loop introduction 32. Additionally, the tip side coupling part 31a of the loop introduction plunger 31 is inserted from the base side of the hollow component for loop introduction 32, and the inner circumferential side of the base side - in short, the outer circumferential side of the tip side coupling part 31a - can be coupled by the base side coupling part 32a. Note that the hollow component for loop introduction 32 is acceptable provided that the inside is hollow. For example, it can be constructed of a round cylinder component (round tube component) or a square cylinder component (square tube component), etc. Further, a slit (or notch) may be formed in a part of the hollow component for loop introduction 32, so as to connect the hollow part of the hollow component for loop introduction 32 and the outside. Additionally, the configuration of the base side coupling part 32a and tip side coupling part 31a is as described with regard to the loop introduction plunger 31.

When the coupled state with the loop introduction plunger 31 is released, the hollow component for loop introduction 32 goes into a state where it can rotate around its axis. Therefore, a lever, etc., may be provided on a part of the side surface of the hollow component for loop introduction 32, and may be made to protrude to outside the housing 50, such that the hollow component for loop introduction 32 can be rotated manually. If configured in this way, the loop forming part 80 coupled with the hollow component for loop introduction 32 can be rotated, and as a result, the orientation of the loop 82 can be appropriately adjusted. Note that a guide for moving the hollow component for loop introduction 32 straight back and forth (for example, a groove into which the hollow component for loop introduction 32 fits) may be formed inside the housing 50.

### Suture Thread Feeding Mechanism 40

The suture thread feeding mechanism 40 is coupled to the movement of the operating part 10, and feeds out suture thread that has been moveably inserted inside the suture thread introduction needle 70 (suture thread 1 shown in FIGS. 1-3) from the tip end (blade edge) of the suture thread introduction needle 70. The suture thread feeding mechanism 40 is constructed from a suture thread introduction plunger 41, a hollow component 42 affixed on the base side of the housing 50, and a suture thread fastening component 43.

The suture thread introduction plunger 41 is coupled to the coupling part 20, and can move in the forward/backward directions inside the housing 50 according to the movement of the operating part 10. The suture thread introduction plunger 41 is such that it moves coupled with the loop introduction plunger 31 according to the movement of the operating part 10. Also, the suture thread introduction plunger 41 has an inner cavity through which the hollow component 42 can pass. In short, the suture thread introduction plunger 41 is configured such that it covers the outside of the hollow component 42. Note that a guide for moving the suture thread introduction plunger 41 straight back and forth (for example, a groove into which the suture thread introduction plunger 41 fits) may be formed inside the housing 50.

The hollow component 42 is formed in a hollow shape and is affixed inside the housing 50. For example, as shown in FIGS. 1-3, the base end 42a of the hollow component 42 may protrude from the base end of the housing 50. The suture thread 1 is passed through from the outside of the housing 50 into the hollow component 42. Here, the suture thread 1 is easy to insert if the base side of the hollow component 42 has a funnel shape (a shape in which the wall surface gradually widens toward the base end). Also, the hollow component 42 may have a length that reaches the base end of the housing 50, and a funnel-shape component having an inner cavity may be attached on the base end of the hollow component 42. Note that the hollow component 42 is acceptable provided that the inside is hollow. For example, it can be constructed of a round cylinder component (round tube component) or a square cylinder component (square tube component), etc. Further, a slit (or notch) may be formed in a part of the hollow component 42, so as to connect the hollow part of the hollow component 42 and the outside.

Also, the length of the hollow component 42 in the axial direction is determined while considering the desired length of the suture thread 1 to protrude from the tip end of the suture thread introduction needle 70. The hollow component 42 is configured in a length such that the tip end does not reach the inner wall surface 54 of the housing 50. In short, the hollow component 42 is configured in a length such that the tip end is opened inside the housing 50. In so doing, the suture thread 1 is exposed inside the housing 50 from the tip end of the hollow component 42 to the inner wall surface 54 of the housing 50, and this exposed length serves as the length of the suture thread 1 fed out from the tip end of the suture thread introduction needle 70.

Additionally, the hollow component 42 may also have the function of guiding the movement of the suture thread introduction plunger 41 in the forward/backward directions. Thus, the axial-direction length of the hollow component 42 is determined while considering not only the length of the desired length of suture thread 1 fed out from the tip end of the suture thread introduction needle 70, but also the length of the suture thread introduction plunger 41. In short, the axial-direction length of the hollow component 42 is configured to a length such that the hollow component 42 is located inside the suture thread introduction plunger 41, even in the state where the suture thread introduction plunger 41 has moved to the forward-most point. As shown in FIG. 3, the axial-direction length of the hollow component 42 is longer than the axial-direction length of the suture thread introduction plunger 41.

Note that, here, for convenience in describing the length of the suture thread 1 fed out from the tip end of the suture thread introduction needle 70, it was described that the suture thread 1 is exposed inside the housing 50 from the tip end of the hollow component 42 to the inner wall surface 54 of the housing 50, but needless to say, a guide tube may be provided as a means for preventing deflection of the suture thread 1, making it so that the suture thread 1 is not exposed (refer to FIG. 9 described below). In this case, the suture thread fastening component 43 does not directly contact the inner wall surface 54 of the housing 50, and forward-direction movement is stopped when the tip side of the component that covers the suture thread fastening component 43 (second guide 402 shown in FIG. 9) contacts a prescribed location of the first guide.

Since the suture thread 1 is passed through the hollow component 42 on the inside, the inside diameter of the hollow component 42 is such that the suture thread 1 can pass through. Also, the hollow component 42 is made so that it can pass through the suture thread fastening component 43. Therefore, the outside diameter of the hollow component 42 must be such that it can pass through the notch in the suture thread fastening component 43. Note that the base end 42a of the hollow component 42 may also protrude outside the housing 50, as shown in FIG. 1. In this case, the burden on the technician of the operation of inserting the suture thread 1 can be greatly reduced. However, it is not mandatory that the base end 42a protrudes outside the housing 50.

The suture thread fastening component 43 is formed of, for example, natural rubber, synthetic rubber, metal, etc., and a notch that penetrates through in the forward/backward directions is formed in the center, for example. The suture thread fastening component 43 can be moved in the forward/backward directions by the suture thread introduction plunger 41. The suture thread fastening component 43 can fasten the hollow component 42, suture thread 1 or suture thread introduction plunger 41 with its notch. In short, depending on the position, which varies according to the movement of the operating part 10, the suture thread fastening component 43 can fasten either the hollow component 42, the suture thread 1 or the suture thread introduction plunger 41. Also, the suture thread fastening component 43 can move in the forward direction until it runs into the inner wall surface 54 of the housing 50, and in the backward direction until it runs into the stopper 51 formed on a part of the inner wall surface on the side surface side of the housing 50.

The suture thread fastening component 43 may be constructed of, for example, an elastic body such as natural rubber or synthetic rubber, or a metal ring of relatively soft metal like copper. If the suture thread fastening component 43 is constructed of an elastic body, it can fasten the suture thread 1 by the elastic force of elastic body. If the suture thread fastening component 43 is constructed of a metal ring, it can fasten the suture thread 1 by plastically deforming at the same time that it comes out of the hollow component 42. For example, if something like a metal ring is used, when the passage inside the housing 50 on the side closer to the tip end than the hollow component 42 (the passage through which the suture thread fastening component 43 moves) is narrowed, the suture thread fastening component 43 moves from the base side, and when it comes out of the hollow component 42, it is spatially caught in the housing 50, and the suture thread fastening component 43 is plastically deformed, and can fasten the suture thread 1. Note that a guide for moving the suture thread fastening component 43 straight back and forth (for example, a groove into which the suture thread fastening component 43 fits) may be formed inside the housing 50. In this case, it is such that the formed guide does not create any frictional resistance against the movement of the suture thread fastening component 43. Also, other than a simple notch, the notch formed in the suture thread fastening component 43 may also be a slit, or a hole having an inside diameter smaller than the outside diameter of the hollow component 42, etc.

### Loop Introduction Needle 60

The loop introduction needle 60 has an inner cavity which houses the loop forming part 80 such that it can move in the forward/backward directions. In the state where the loop introduction needle 60 is attached to the housing 50, its base end opens inside the housing 50, such that it connects through to the inside of the housing 50. The loop introduction needle 60 is attached to the tip side of the housing 50 such that its axial center aligns with the axial center of the loop feeding/returning mechanism 30. If the loop introduction needle 60 is attached to the housing 50 in this manner, it can feed and return the loop forming part 80 in a linear manner. The loop introduction needle 60 may be formed of a metal such as stainless steel, for example.

Also, the loop introduction needle 60 has a blade edge for puncturing the skin at the tip end. This blade edge may be formed such that it is sectioned on a plane that intersects the axial center of the loop introduction needle 60 at an angle. In order that the loop 82 of the loop forming part 80 described later reliably extends in the direction of the suture thread introduction needle 70, the tip opening of the loop introduction needle 60 may face toward the suture thread introduction needle 70.

Note that the loop introduction needle 60 is acceptable provided that it can puncture the skin and insert the loop forming part 80. For example, as the loop introduction needle 60, something that has an outside diameter of about 21-17 G (preferably 20-18 G) and a length of 70-120 mm (preferably 80-100 mm) may be used. Also, at the tip end of the loop introduction needle 60, a beveled part may be formed so as not to cut the grasped suture thread 1. Additionally, the position of the tip end of the loop introduction needle 60 (the position facing the fed-out suture thread 1) may be made adjustable in accordance with the position of the supporting part 52.

### Suture Thread Introduction Needle 70

The suture thread introduction needle 70 has an inner cavity, which houses the suture thread 1 such that it can move in the forward/backward directions. When the suture thread introduction needle 70 is attached to the housing 50, the base opens inside the housing 50, so as to connect through to the inside of the housing 50. The suture thread introduction needle 70 is attached on the base side of the housing 50 so that its axial center aligns with the axial center of the suture thread feeding mechanism 40. If the suture thread introduction needle 70 is attached to the housing 50 in this manner, it can feed out the suture thread 1 in a linear manner. The suture thread introduction needle 70 may be formed of a metal such as stainless steel, for example.

Also, the suture thread introduction needle 70 has a blade edge for puncturing the skin at the tip end. This blade edge may be formed such that it is sectioned on a plane that intersects the axial center of the suture thread introduction needle 70 at an angle. The orientation of the tip opening of the suture thread introduction needle 70 is not particularly limited, but it may be oriented in the direction of the loop introduction needle 60.

Note that the shape of the suture thread introduction needle 70 is not particularly limited, provided that it can puncture the skin and insert the suture thread 1. For example, as the suture thread introduction needle 70, something that has an outside diameter of about 21-17 G (preferably 20-18 G) and a length of 70-120 mm (preferably 80-100 mm) may be used. The distance between the loop introduction needle 60 and the suture thread introduction needle 70 may be set to the length which allows the suture thread 1 to affix the abdominal wall and visceral organ wall of the patient (for example, about 10-30 mm). If the distance between the loop introduction needle 60 and the suture thread introduction needle 70 is in this range, the abdominal wall and visceral organ wall of the patient can be sufficiently affixed.

### Loop Forming Part 80

The loop forming part 80 is inserted inside the loop introduction needle 60 such that it can move in the forward/backward directions. The loop forming part 80 is constructed of a base side affixing part 83 affixed to the tip end of the hollow component for loop introduction 32, a loop 82 affixed to the tip end, and a rod-like shaft 81 having an outside diameter smaller than the inside diameter of the loop introduction needle 60, which connects the base side affixing part 83 and the loop 82. The loop 82 is formed by an elastic material, and in the state where it has been fed out from the tip end of the loop introduction needle 60 (the state where no external force is applied), it is restored to a ring shape, and in the state where it has not been fed out from the tip end of the loop introduction needle 60, it can be housed inside the loop introduction needle 60 while varying substantially linearly.

In the state where the loop 82 has been fed out from the tip end of the loop introduction needle 60, it extends in the direction of the suture thread introduction needle 70 such that the suture thread 1 fed out from the tip end of the suture thread introduction needle 70 passes through the inside of the loop 82. For example, the loop 82 may be shaped such that it is affixed to the tip end of the rod-like shaft 81 at a prescribed angle, and takes on a curved shape in the state where it has been fed out from the tip end of the loop introduction needle 60. For example, when the loop 82 is viewed from the side surface, the curved shape may be such that the apex portion of the curved portion protrudes in the forward direction. If the loop 82 is formed with such a shape, the suture thread 1 can be more reliably positioned inside the loop 82. Also, if the center of the loop 82 emerges upon elongation of the suture thread introduction needle 70, the suture thread 1 can be more reliably positioned inside the loop 82 when the suture thread 1 is pushed straight.

Also, the tip end shape of the loop 82 is not particularly limited, but it may be substantially V-shaped or substantially U-shaped centered around the tip end, so as to reduce the distance of the portion that grasps the suture thread (the portion that is substantially V shaped or substantially U shaped). By so doing, the suture thread 1 fed out from the tip end of the suture thread introduction needle 70 can be more reliably grasped. The loop 82 may be constructed of any deformable material. For example, it may be constructed of stainless steel wire (high tensile strength stainless steel used for springs), piano wire (nickel-plated or chromium-plated piano wire), or super-elastic alloy wire (titanium-nickel alloy, copper-zinc alloy (or those alloys also containing beryllium, silicon, tin, aluminum, gallium, etc.), nickel-aluminum alloy, etc.).

The rod-like shaft 81 may be constructed using metal (for example, stainless steel) or a synthetic resin (for example, polyolefins such as polypropylene and polyethylene, and fluorine resins such as PTFE and ETFE), etc. The rod-like shaft 81 may be constructed using a stylet, etc. Also, the base side affixing part 83 is acceptable provided that it is affixed to the tip end of the hollow component for loop introduction 32, and a notch, etc., may be provided on the tip end of the hollow component for loop introduction 32, such that the base side affixing part 83 is affixed by fitting into that notch. Note that if the loop 82 is constructed of a relatively rigid material, the rod-like shaft 81 and the loop 82 may be constructed of the same material. In this case, the rod-like shaft 81 and the loop 82 may be constructed in an integral manner or as separate bodies.

### Other Constituent Parts

Although not shown in the drawings, the suturing implement 100 may have a flat plate part through which the loop introduction needle 60 and suture thread introduction needle 70 pass. The flat plate part moves toward the tip side when the loop introduction needle 60 and suture thread introduction needle 70 puncture the skin, thereby suppressing any change in distance between the loop introduction needle 60 and suture thread introduction needle 70. By a flat plate part being provided in this manner, the distance between the loop introduction needle 60 and suture thread introduction needle 70 can be prevented from changing a large amount. Also, the flat plate part does not hinder the puncture operation if it is able to move in the forward/backward directions with respect to the loop introduction needle 60 and suture thread introduction needle 70.

The flat surface shape of the flat plate part is not particularly limited, but it may be, for example, a rectangle, circle or polygon. Also, the tip side surface of the flat plate part (surface on patient side) may be flat so as not to irritate the patient's skin. When such a flat plate part is provided, the tip ends of a pair of support rods, which extend in parallel with the loop introduction needle 60 and suture thread introduction needle 70, may be affixed to the flat plate part. By providing support rods, the movement stability of the flat plate part can be improved, and force transmitted from the flat plate part to the loop introduction needle 60 and suture thread introduction needle 70 can be reduced.

An elastic component such as a spring or rubber may be provided in the guide hole 55, such that the operating part 10 can be moved in the backward direction utilizing the rebound force of the elastic component. If such an elastic component is provided, the operation when moving the operating part 10 in the backward direction is easier, and the burden of operation on the technician is further reduced. In short, the suture thread 1 can be passed through the visceral organ wall and abdominal wall with a substantially one-touch operation, without the technician performing the returning operation described below.

### Operation of Suturing Implement 100

As preparatory operations of the suturing implement 100, the technician first inserts the suture thread 1 into the hollow component 42 of the suture thread feeding mechanism 40. At this time, the technician feeds the suture thread 1 into the suture thread introduction needle 70 to a length such that the suture thread 1 does not protrude from the tip end of the suture thread introduction needle 70. After the suturing implement 100 has been stuck into the visceral organ, when the operating part 10 is moved forward in this state, the movement of the operating part 10 is transferred to the loop introduction plunger 31 and suture thread introduction plunger 41 via the coupling part 20. Also, the loop introduction plunger 31 and suture thread introduction plunger 41 start to move forward. Note that the technician may also use the suturing implement 100 in the state where the suture thread 1 has been set in the suturing implement 100 in advance.

### Operation of Loop Feeding/Returning Mechanism 30

Due to the fact that the loop introduction plunger 31 and hollow component for loop introduction 32 are in the state where they are coupled with the operating part 10 via the coupling part 20, the loop introduction plunger 31 and hollow component for loop introduction 32 move in a coupled manner according to movement of the operating part 10. Since the loop forming part 80 is joined to the tip end of the hollow component for loop introduction 32, the loop forming part 80 also moves forward accompanying forward-direction movement of the hollow component for loop introduction 32. When this occurs, the loop 82 located at the tip end of the loop forming part 80 is fed out from the tip end of the loop introduction needle 60 and opens into a ring shape.

Additionally, when the operating part 10 is moved in the forward direction, the hollow component for loop introduction 32 runs into the inner wall surface 54 of the housing 50. In this state, when the operating part 10 is moved further forward, the coupled state of the loop introduction plunger 31 and hollow component for loop introduction 32 is released, and only the loop introduction plunger 31 moves forward. That is, the loop 82 that was fed out from the tip end of the loop introduction needle 60 can be maintained in the open state as is. By this configuration, the timing of feed-out of the suture thread 1 can be made later than the timing of feed-out of the loop 82. That is, by the suturing implement 100, a time differential can be provided between the timing of feed-out of the loop 82 and the timing of feed-out of the suture thread 1.

Then, when the operating part 10 is moved backward, the loop introduction plunger 31 also starts to move backward. When the tip side coupling part 31a couples with the base side coupling part 32a, the hollow component for loop introduction 32 also starts to move backward. When the hollow component for loop introduction 32 starts to move backward, the loop 82 also starts to move backward. At this time, the suture thread 1 fed out from the tip end of the suture thread introduction needle 70 passes through the loop 82. Therefore, when the loop 82 starts to move backward, the ring shape of the loop 82 gradually becomes smaller, and the suture thread 1 is grasped by the loop 82. Further, when the loop 82 moves backward, the suture thread 1 is grasped by the tip end of the loop introduction needle 60.

### Operation of Suture Thread Feeding Mechanism 40

In accordance with the movement of the operating part 10, the suture thread introduction plunger 41 also starts to move at the same time as movement of the loop introduction plunger 31. When the suture thread introduction plunger 41 moves forward, the suture thread fastening component 43 is pushed by the tip end of the suture thread introduction plunger 41. When in the initial state (or the state just before the second operation or subsequent operations), the suture thread fastening component 43 is in a state where it gets stuck in the hollow component 42, and by being pushed by the tip end of the suture thread introduction plunger 41, it moves forward along the top of the hollow component 42. In the state where the hollow component 42 has been inserted through the notch of the suture thread fastening component 43 - that is, in the state where the suture thread fastening component 43 has gotten stuck in the hollow component 42 and has fastened the hollow component 42 - the fastening force of the suture thread fastening component 43 does not reach the suture thread 1, and the suture thread 1 becomes free (the state where the suture thread 1 is not fastened to the suture thread fastening component 43).

When the suture thread introduction plunger 41 moves in the forward direction, the suture thread fastening component 43 also moves forward, and the suture thread fastening component 43 comes out from the hollow component 42. In the state where the suture thread 1 has been inserted through the notch of the suture thread fastening component 43 - in short, in the state where the suture thread fastening component 43 has fastened the suture thread 1 - the fastening force of the suture thread fastening component 43 reaches the suture thread 1, and the suture thread 1 is grasped by the suture thread fastening component 43. That is, when the suture thread fastening component 43 is pushed forward by the suture thread introduction plunger 41 and the suture thread fastening component 43 comes out of the hollow component 42, the fastened state of the hollow component 42 by the suture thread fastening component 43 is released, and it results in the state where the suture thread 1 is fastened by the suture thread fastening component 43. In this state, if the suture thread introduction plunger 41 is moved further forward, the suture thread 1 grasped by the suture thread fastening component 43 moves forward together with the suture thread fastening component 43.

By the suture thread 1 moving forward, the suture thread 1 is fed out from the tip end of the suture thread introduction needle 70. Since the loop 82 is fed out from the tip end of the loop introduction needle 60 sooner than the suture thread 1 is fed out, it easily reaches the state where the suture thread 1 has been inserted inside the loop 82.

In addition, when the suture thread introduction plunger 41 moves forward, the suture thread fastening component 43 runs into the inner wall surface 54 of the housing 50, and movement is stopped. In this state, if the suture thread introduction plunger 41 moves further forward, the suture thread introduction plunger 41 sticks into the suture thread fastening component 43. Thus, the fastening of the suture thread fastening component 43 to the suture thread 1 is released, and the suture thread 1 becomes free. In short, the suture thread 1 goes into a state where it does not move forward. In the state where the suture thread introduction plunger 41 has been stuck into the notch of the suture thread fastening component 43, the suture thread fastening component 43 moves in accordance with the backward-direction movement of the suture thread introduction plunger 41.

Next, when the operating part 10 is moved in the backward direction, the suture thread introduction plunger 41 also moves backward. Also, the suture thread fastening component 43 moves backward together with the suture thread introduction plunger 41 until it contacts - that is, runs into - the stopper 51. In the state where the suture thread fastening component 43 has contact the stopper 51, if the suture thread introduction plunger 41 is moved further backward, movement of the suture thread fastening component 43 is stopped by the stopper 51, and as a result, the suture thread introduction plunger 41 comes out from the notch of the suture thread fastening component 43, and only the suture thread introduction plunger 41 moves backward. In short, the fastening force of the suture thread fastening component 43 is applied to the hollow component 42 from the suture thread introduction plunger 41, and returns it to the initial state.

Via this series of operations, each part (the operating part 10, coupling part 20, loop feeding/returning mechanism 30, suture thread feeding mechanism 40) returns to the same position as its initial state, but the loop 82 goes into the state where it is grasping the suture thread 1. In this state, when the loop introduction needle 60 and suture thread introduction needle 70 of the suturing implement 100 come out from the visceral organ on the abdominal wall side, the suture thread 1 becomes free, and therefore, the suture thread 1 is guided smoothly, and goes into the state where it has passed through the visceral organ wall and abdominal wall in a substantially U shape. Finally, the suture thread 1 is cut by the blade edge of the suture thread introduction needle 70 which has been pulled outside the body or by another cutting means (scissors or cutter, etc.). When this is done, the suture thread 1 is in the same state as the initial state, where it has been inserted up to the tip end of the suture thread introduction needle 70, and therefore, the visceral organ wall can be affixed by performing the above procedure any number of times. Note that after the suture thread 1 is cut, if the suture thread 1 emerges on the tip side beyond the blade edge of the suture thread introduction needle 70, it can be put in the same state as the initial state by pulling the suture thread 1 by hand on the base side.

### Feed-out Timing of Loop 82 and Suture Thread 1

According to the suturing implement 100, movement of the operating part 10 is first transferred to the loop 82, and after that, movement of the operating part 10 is transferred to the suture thread 1. As a result, the timing of feed-out of the suture thread 1 can be made later than the timing of feed-out of the loop 82. By so doing, the suture thread 1 can be reliably inserted inside the loop 82, and the suture thread 1 can be reliably grasped.

Specifically, the loop feeding/return mechanism 30 and the suture thread feeding mechanism 40 both start to operate coupled with the movement of the operating part 10, but on the loop feeding/returning mechanism 30 side, the movement of the operating part 10 is transferred to the loop 82 using the coupling of the loop introduction plunger 31 and hollow component for loop introduction 32, and on the suture thread feeding mechanism 40 side, the suture thread 1 is made to remain free by utilizing the state where the suture thread fastening component 43 has gotten stuck in the hollow component 42, so that only the loop 82 is first fed out. Then, in order that the suture thread 1 is fed out in the state where the loop 82 has been completely fed out, on the loop feeding/returning mechanism 30 side, the coupled state of the loop introduction plunger 31 and hollow component for loop introduction 32 is released, the movement of the operating part 10 is not transferred to the loop 82, and the shape of the loop 82 can be maintained, and on the suture thread feeding mechanism 40 side, the suture thread fastening component 43 comes out from the hollow member 42 and fastens the suture thread 1, and the movement of the operating part 10 is transferred to the suture thread 1.

In this way, in the suturing implement 100, a time differential can be provided between the feed-out timing of the loop 82 and the feed-out timing of the suture thread 1. Specifically, in the suturing implement 100, the suture thread 1 can be fed out in the state where the loop 82 has been fed out beforehand, and the suture thread is easily inserted inside the loop 82 formed in a ring shape. Therefore, according to the suturing implement 100, the feed-out timing of the suture thread 1 and the feed-out timing of the loop 82 can be changed or corrected by adjusting the position of the stopper 51 as well as the lengths of the loop introduction plunger 31, hollow component for loop introduction 32, suture thread introduction plunger 41 and hollow component 42.

### Specific Operating Procedure of Suturing Implement 100

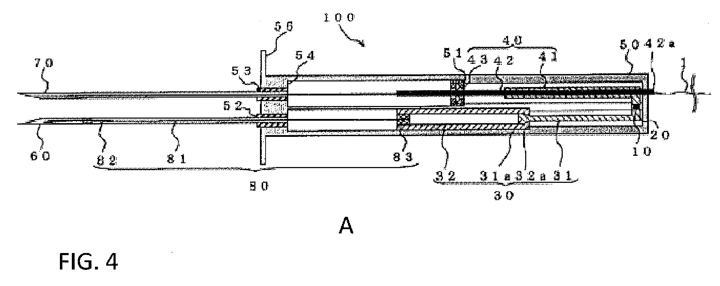

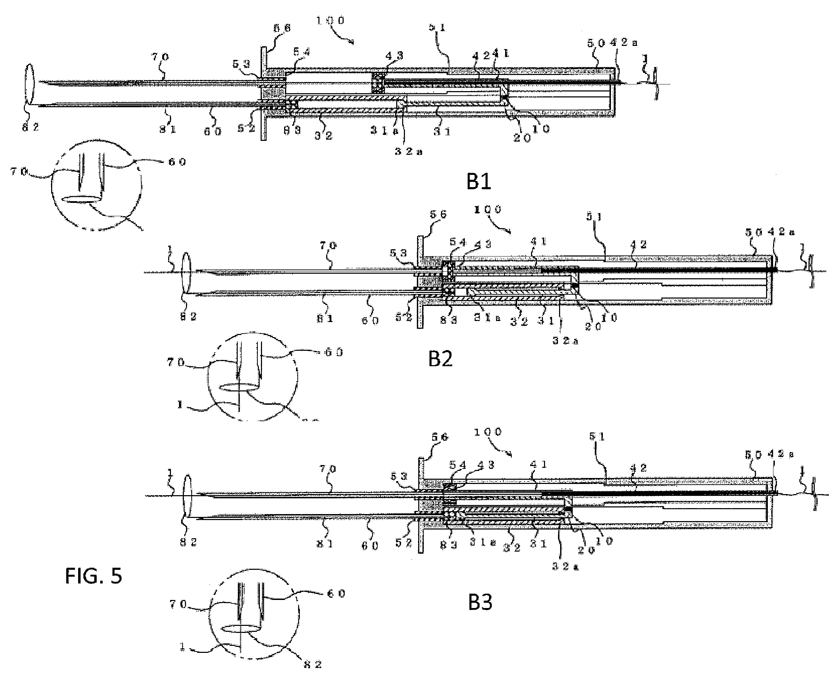

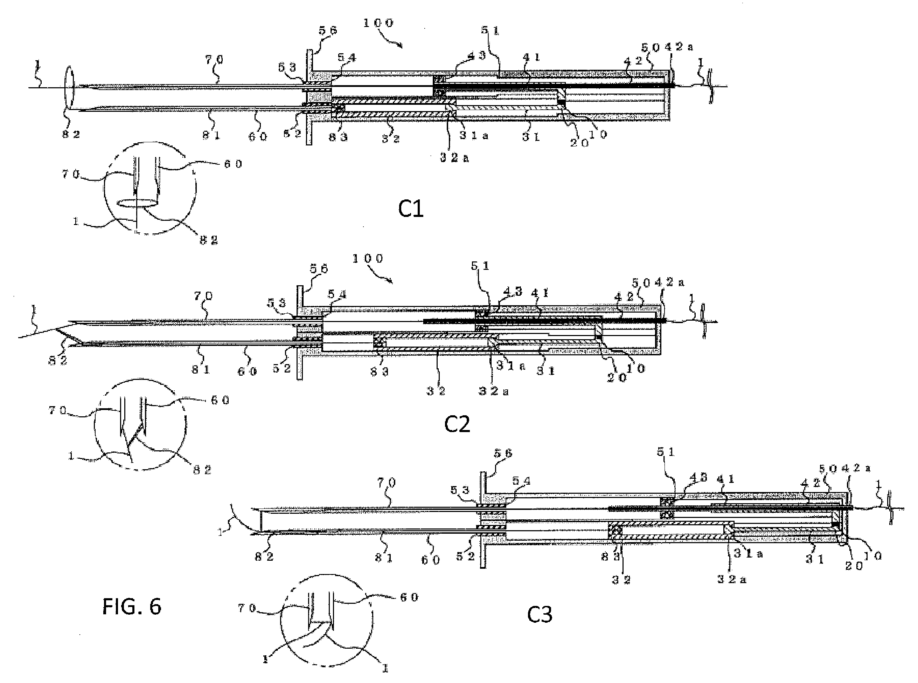

FIGS. 4-6 are schematic cross-sectional views which illustrate the operating procedure of the suturing implement 100 in a simplified manner. The specific operating procedure of the suturing implement 100 will be described based on FIGS. 4-6. FIG. 4 illustrates the puncturing operation of the suturing implement 100, FIG. 5 illustrates the forward-direction movement operation of each part (called the "plunging operations" (plunging operation 1 (B1)-plunging operation 3 (B3)) hereinafter), and FIG. 6 illustrates the backward-direction movement operation of each part (called the "returning operations" (returning operation 1 (C1)-returning operation 3 (C3)) hereinafter). FIGS. 5 and 6 also show inset views of the feed-out situation of the suture thread 1 and loop 82.

### (A) Puncturing (when in the initial state of the suturing implement 100 (including the state just before the second operation and subsequent operations))

First, with the suturing implement 100 in the state where the loop 82 of the loop forming part 80 is housed inside the loop introduction needle 60 and the suture thread 1 is housed inside the suture thread introduction needle 70, the technician sticks the suturing implement 100 into the patient. At this time, the suture thread 1 is housed inside the suture thread introduction needle 70 so as not to protrude from the suture thread introduction needle 70. Also, the suture thread fastening component 43 is in the state where it has gotten stuck in the hollow component 43, and the suture thread 1 is free - in short, the state where it does not move in the forward direction. Further, the loop introduction plunger 31 and hollow component for loop introduction 32 are in the coupled state.

### (B1) Plunging operation 1 (from start of feed-out of loop 82 to start of feed-out of suture thread 1)

When forward-direction force is applied to the operating part 10 by the technician, the loop introduction plunger 31 of the loop feeding/returning mechanism 30 and the suture thread introduction plunger 41 of the suture thread feeding mechanism 40 start to move forward via the coupling part 20. Then, after the suture thread introduction plunger 41 contacts the suture thread fastening component 43, the suture thread fastening component 43 pushed by the suture thread introduction plunger 41 moves in the forward direction by sliding along the hollow component 42. The suture thread 1 remains free. Accompanying the movement of the loop introduction plunger 31, the hollow component for loop introduction 32 which is coupled with the loop introduction plunger 31 also starts to move forward.

When the loop introduction plunger 31 moves in the forward/backward directions in the coupled state, the hollow component for loop introduction 32 also moves in the forward/backward directions together with the loop introduction plunger 31. The hollow component for loop introduction 32 can move forward until the tip surface runs into the inner wall surface 54 of the housing 50. When the hollow component for loop introduction 32 moves forward until the tip surface runs into the inner wall surface 54 of the housing 50, the loop forming part 80 connected to the tip end of the hollow component for loop introduction 32 also moves the inner cavity of the loop introduction needle 60 to the tip side, and is fed out from the tip end of the loop introduction needle 60. When the loop 82 is fed out from the tip end of the loop introduction needle 60, it results in a state where external force is not applied, and it is restored to a ring shape.

Then, when the operating part 10 is again moved in the forward direction by the technician, the loop introduction plunger 31 and the suture thread introduction plunger 41 each continue to move forward, and the suture thread fastening component 43 pushed by the suture thread introduction plunger 41 comes out from the hollow component 42, so as to fasten the suture thread 1.

### (B2) Plunging operation 2 (feed-out of suture thread 1 complete)

In this state, when the suture thread fastening component 43 moves further forward, the suture thread 1 fastened to the suture thread fastening component 43 also starts to move forward, and the suture thread 1 starts to be fed out from the tip end of the suture thread introduction needle 70. Then, the suture thread 1 passes through the inside of the loop 82 that had already been fed out. Meanwhile, the hollow component for loop introduction 32 runs into the inner wall surface 54 of the housing 50, and the coupling with the loop introduction plunger 31 is released. Therefore, only the loop introduction plunger 31 moves forward in the state where the shape of the loop 82 has been maintained.

That is, when the tip surface of the hollow component for loop introduction 32 runs into the inner wall surface 54 of the housing 50, forward-direction movement of the hollow component for loop introduction 32 is restricted, but by the operating part 10 being moved further forward, the coupled state of the base side coupling part 32a and the tip side coupling part 31a is released, and only the loop introduction plunger 31 moves forward. The hollow component for loop introduction 32 does not move further toward the tip side than that, and only the loop introduction plunger 31 can move toward the tip side. As a result, the shape of the loop 82 fed out from the tip end of the loop introduction needle 60 is maintained.

### (B3) Plunging operation 3 (release of fastening of fed-out suture thread 1)

When the operating part 10 is moved further forward by the technician, the loop introduction plunger 31 and the suture thread introduction plunger 41 each continue to move forward. However, the suture thread fastening component 43 pushed by the suture thread introduction plunger 41 runs into the inner wall surface 54 of the housing 50 and cannot move further forward than that. In this state, when the suture thread introduction plunger 41 moves further forward, the suture thread introduction plunger 41 is stuck into the suture thread fastening component 43. When this occurs, the fastening of the suture thread 1 is released, and the suture thread 1 again becomes free. In short, the suture thread 1 no longer moves in the forward direction. Meanwhile, on the loop feeding/returning mechanism 30 side, only the loop introduction plunger 31 moves forward in the state where the shape of the loop 82 is maintained. Therefore, the state where the suture thread 1 has passed through the inside of the loop 82 is maintained.

### (C1) Returning operation 1 (start of backward-direction movement of each part)

When backward-direction force is applied to the operating part 10 by the technician, the loop introduction plunger 31 of the loop feeding/returning mechanism 30 and the suture thread introduction plunger 41 of the suture thread feeding mechanism 40 start to move backward via the coupling part 20. At this time, the suture thread fastening component 43, in the state where the suture thread plunger 41 has been stuck into it, moves backward together with the suture thread introduction plunger 41. The suture thread 1 remains free. Also, the loop introduction plunger 31 moves the inside of the hollow component for loop introduction 32 backward. Then, in the state where the tip end of the loop introduction plunger 31 has reached the base end of the hollow component for loop introduction 32, when further backward-direction force is applied to the operating part 10, the loop introduction plunger 31 and the hollow component for loop introduction 32 are again coupled.

### (C2) Returning operation 2 (start of return of loop 82)

When the operating part 10 is moved further backward by the technician, the loop introduction plunger 31 and the suture thread introduction plunger 41 each continue to move backward. However, the backward-direction movement of the suture thread fastening component 43 is restricted by the stopper 51, and the suture thread introduction plunger 41 comes out from the suture thread fastening component 43. When this occurs, the suture thread fastening component 43 goes into a state where it has gotten stuck in the hollow component 42, the same as the initial state. The suture thread 1 remains free. In short, the suture thread 1 does not move.

Meanwhile, when the hollow component for loop introduction 32 moves backward due to being coupled with the loop introduction plunger 31, the loop forming part 80 joined with the hollow component for loop introduction 32 also starts to move backward. When this occurs, the loop 82 starts to be housed inside the loop introduction needle 60. At this time, since the suture thread 1 has passed through inside the loop 82, the loop 82 pulls the suture thread 1 toward the loop introduction needle 60 side.

### (C3) Returning operation 3 (grasping of suture thread 1 complete)

When the operating part 10 is moved further backward by the technician, the loop introduction plunger 31 and the suture thread introduction plunger 41 each continue to move backward. However, on the suture thread feeding mechanism 40 side, only the suture thread introduction plunger 41 moves backward. On the other hand, accompanying the backward-direction movement of the hollow component for loop introduction 32, the loop 82 pulls the suture thread 1 toward the tip end of the loop introduction needle 60, and finally, the suture thread 1 is grasped by the tip end of the loop introduction needle 60. At this time, each part returns to the same position as the initial state, but the loop 82 is in the state where it is grasping the suture thread 1.

In this state, when the suturing implement 100 is removed, the suture thread 1 becomes free, and therefore, the suture thread 1 is guided smoothly, and goes into the state where it has passed through the visceral organ wall and abdominal wall in a substantially U shape. In short, by the technician simply performing the plunging operations and returning operations, the suture thread 1 can be put into a state where it has passed through the visceral organ wall 102 and abdominal wall 101 in a substantially U shape. Finally, when the suture thread 1 is cut by the blade edge of the suture thread introduction needle 70 which has been pulled outside the body or by another cutting means, the suture thread 1 is in the same state as the initial state, where it has been inserted up to the tip end of the suture thread introduction needle 70. As a result, the visceral organ wall can be affixed by performing the above procedure any number of times. Note that after the suture thread 1 is cut, if the suture thread 1 emerges on the tip side beyond the blade edge of the suture thread introduction needle 70, it can be put in the same state as the initial state by pulling the suture thread 1 by hand on the base side.

### Organ Fixing Procedure Using Suturing Implement 100

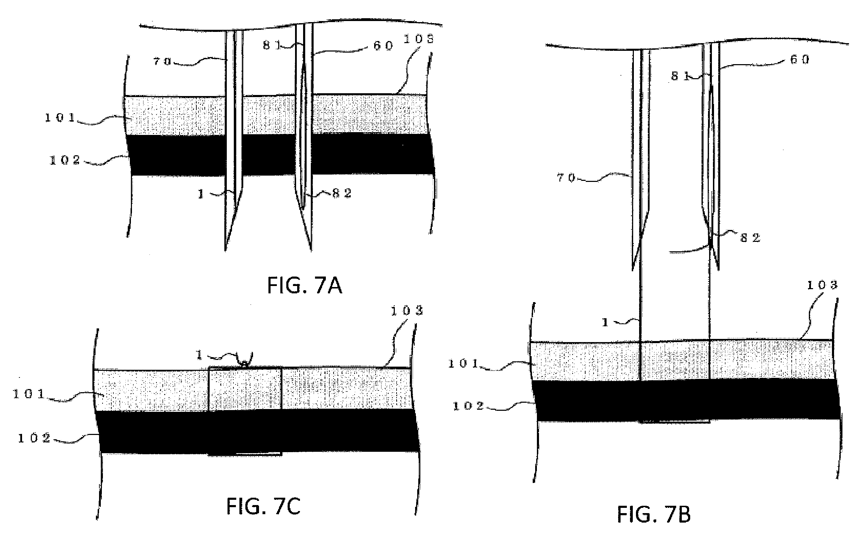

FIG. 7 is a schematic diagram for explaining the operation of the suturing implement 100. The operation of the suturing implement 100 will be explained based on FIG. 7 following the flow of the procedure using the suturing implement 100. Here, the case where the abdominal wall 101 and visceral organ wall 102 (for example, gastric wall) of a patient are affixed using the suturing implement 100 is explained.

First, the technician inserts an endoscope inside the visceral organ (for example, the stomach) from the mouth or nose of the patient. After that, the technician supplies sufficient gas (for example, carbon dioxide) into the visceral organ to expand the visceral organ. Then, he seals the visceral organ wall 102 to the abdominal wall 101 in an airtight manner. After that, he disinfects the skin 103, including the locations to be punctured by the loop introduction needle 60 and the suture thread introduction needle 70. After that, he checks the position of the visceral organ by means of light emitted from the endoscope, and performs local anesthesia at the site.

Then, the technician prepares the suturing implement such that the loop 82 of the loop forming part 80 is housed inside the loop introduction needle 60, and the suture thread 1 is housed inside the suture thread introduction needle 70 (the state illustrated in FIG. 4). At this time, the suture thread 1 is housed inside the suture thread introduction needle 70 so as not to protrude from the tip end of the suture thread introduction needle 70. Then, the technician sticks the loop introduction needle 60 and suture thread introduction needle 70 of the suturing implement 100 into the abdominal wall 101 of the patient, causing the loop introduction needle 60 and suture thread introduction needle 70 to protrude from the visceral organ wall 102 into the visceral organ (FIG. 7A).

After he confirms this state with an endoscope, the technician moves the operating part 10 forward. Then, the technician executes the series of operations shown in FIG. 5 and FIG. 6, putting the suture thread 1 in the state where it is exposed outside the body (FIG. 7B). In short, the suture thread 1 goes into a state where it has passed through the visceral organ wall 102 and abdominal wall 101 in a substantially U shape. Finally, the technician cuts the suture thread that was pulled outside the body, and ligates the two ends of the suture thread 1 (FIG. 7C). By this ligation, the visceral organ wall 102 and abdominal wall 101 are affixed.

Further, the suturing implement 100 is again stuck in at a location substantially parallel to and separated by a prescribed distance - for example, 20-30 mm - from the ligated portion. In short, by means of the suture thread 1 functioning as an organ fixing implement, the visceral organ wall 102 and abdominal wall 101 are affixed, and the fistula used when a fistula catheter is inserted can be easily formed. At this time, if the length of the suture thread 1 is such that it can be used a plurality of times, the suturing implement 100 can be used continuously.

### Effects Exhibited by the Suturing Implement 100

According to the suturing implement 100, it is unnecessary to insert suture thread 1 into the suturing implement 100 each time, and organ fixing is possible any number of times until there is no more suture thread 1. Also, according to the suturing implement 100, the suture thread 1 can be put into the state where it has passed through the visceral organ wall 102 and abdominal wall 101 in a substantially U shape, simply by push-pull operation of the operating part 10 (operations (A)-(C) above). Specifically, simply by the technician executing the operation of moving the operating part 10 forward and the operation of moving the operating part 10 backward, the suture thread 1 can be put into the state where it has passed through the visceral organ wall 102 and abdominal wall 101, and therefore the procedure required by the technician is made much simpler. In addition, the suture thread 1 can be reliably inserted inside the loop 82 because the suture thread 1 is fed out after the loop 82 goes into the state where it is open in a ring shape.

Therefore, the burden of operation on the technician can be greatly reduced, and manipulability is markedly improved. Also, since the procedure itself can be executed very easily, not only manipulability but also operability is markedly improved. In addition, even while the procedure is simple, grasping of the suture thread 1 can be performed reliably, and therefore the need to puncture multiple times can be dramatically reduced, which contributes to reducing not only the burden on the technician, but the burden on the patient as well.

Another Example of Configuration of Suture Thread Feeding Mechanism
FIG. 9 is a schematic view illustrating an another example of the configuration of the suture thread feeding mechanism of the suturing implement 100 (called "suture thread feeding mechanism 400" hereinafter). The configuration of the suture thread feeding mechanism 400 will be described based on FIG. 9. The suture thread feeding mechanism 400 is configured such that the suture thread 1 is not exposed inside the housing 50. Also, the suture thread fastening component 43 may be constructed from an elastic body or metal ring as described above, but it may also be covered by a separate component (for example, the second guide 402 shown in FIG. 9). Note that FIG. 9 shows an enlargement of the suture thread feeding mechanism 400 of the suturing implement 100. Also, FIG. 9 illustrates only the side nearest the suture thread feeding mechanism 400, but a guide tube may also be similarly provided on the side nearest the loop feeding/returning mechanism.

As shown in FIG. 9, a first guide 401 which protrudes from the tip side toward the base side is formed inside the housing 50. This first guide 401 has an inner cavity, through which the second guide 402 passes. In short, the first guide 401 guides movement of the suture thread fastening component 43 in the forward/backward directions together with the second guide 402. Also, the inner cavity of the first guide 401 connects through to the inner cavity of the suture thread introduction needle 70. In addition, the tip side of the inner cavity of the first guide 401 is formed in a tapered shape which gradually widens from the tip side toward the base side, such that the tip end of the second guide 402 contacts a part of the tip side.

The suture thread fastening component 43 is covered by the second guide 402. The tip side of the second guide 402 has an outside diameter that is smaller than the inside diameter of the first guide 401, such that it protrudes toward the tip side. Also, the second guide 402 moves accompanying the forward/backward-direction movement of the suture thread fastening component 43, and is able to be inserted into the inner cavity of the first guide 401. Forward-direction movement of the second guide 402 is stopped due to the fact that the tip end contacts the tip side on the part where the first guide 401 is formed in the tapered shape. Note that forward-direction movement of the second guide 402 may also be stopped by the base end of the first guide 401 hitting a prescribed position on the second guide 402 which is covered by the suture thread fastening component 43 (for example, a part on the outer wall surface of the first guide 402 [*sic*]).

When the suture thread fastening component 43 is pushed by the suture thread introduction plunger 41, the suture thread fastening component 43 moves forward, but accompanying this, the second guide 402 is guided in the first guide 401 while moving in the forward direction. When the tip end of the second guide 402 contacts the prescribed location of the first guide 401 (for example, the inner wall surface at the portion where it is formed in a tapered shape), forward-direction movement is restricted, and forward-direction movement beyond this is stopped. The stop position of the second guide 402 may be determined according to the feed-out length of the suture thread 1.

Note that here, an example was described in which a separate component that covers the suture thread fastening component 43 is made to function as a guide, but the configuration is not limited thereto, and a guide mechanism may or may not be provided by a component that covers the suture thread fastening component 43. Also, needless to say, a configuration in which the suture thread fastening component 43 is covered by a separate component may also be similarly applied to a second embodiment, and not just the first embodiment.

FIG. 8 is a schematic interior configuration view illustrating an example of the interior configuration of the medical suturing implement according to embodiment 2 of the present invention (called simply "suturing implement 100A" hereinafter). Similar to the suturing implement 100 according to embodiment 1, the suturing implement 100A is used when introducing an organ fixing implement, used in affixing the abdominal wall and visceral organ wall which is performed in order to make insertion of a fistula catheter easy, into the visceral organ. Note that embodiment 2 is described while focusing on the differences from embodiment 1, and the parts that are the same as embodiment 1 are assigned the same reference numerals, and their described is omitted.

The suturing implement 100 according to embodiment 1 assumes repeated use, but the suturing implement 100A according to embodiment 2 does not assume repeated use. That is, the suturing implement 100 according to embodiment 1 is such that suture thread 1 having a length that corresponds to a plurality of procedures is inserted, but the suturing implement 100A according to embodiment 2 is such that suture thread 1 having a length that corresponds to one procedure is inserted. Accordingly, the constituent parts of the suturing implement 100A can be simplified. It is described specifically below.

The suturing implement 100A does not assume repeated use. For this reason, first, the length of the suture thread 1 is only the length for one procedure. Also, since there is no need to re-insert the suture thread fastening component 43 that came out from the hollow component 42 back into the hollow component 42, the stopper 51 is unnecessary. Additionally, since it is also possible to fasten the suture thread with the suture thread fastening component 43 in advance, the hollow component 42 is unnecessary.

According to the suturing implement 100A, there is no need to adjust the suture thread 1 to the appropriate position each time, thereby reducing the burden of that portion of the operation. Also, according to the suturing implement 100A, simply by plunging operations (plunging operations described in embodiment 1 (plunging operation 1 (B1)-plunging operation 3 (B3))) and returning operations (returning operations described in embodiment 1 (returning operation 1 (C1)-returning operation 3 (C3))) of the operating part 10, the suture thread 1 can be put into a state where it has passed through the visceral organ wall 102 and abdominal wall 101 in a substantially U shape. Specifically, simply by the technician executing the operation of moving the operating part 10 in the forward direction, the suture thread 1 can be put into a state where it has passed through the visceral organ wall 102 and abdominal wall 101, and therefore, the procedure required of the technician is greatly simplified. Additionally, the suture thread 1 can be reliably inserted inside the loop 82 because the suture thread 1 is fed out after the loop 82 has been opened into a ring shape.

Therefore, the burden on the technician is greatly reduced, and manipulability is markedly improved. Also, since the procedure itself can be executed very easily, not only manipulability but also operability is markedly improved. In addition, even while the procedure is simple, grasping of the suture thread 1 can be performed reliably, and therefore the need to puncture multiple times can be dramatically reduced, which contributes to reducing not only the burden on the technician, but the burden on the patient as well.

Note that in the case where the hollow component 42 is not used, the movement of the suture thread fastening component 43 is the same as the movement from the tip-most position of the hollow component 42 in the case where the hollow component 42 is used as shown in FIG. 8. Thus, because there is a guide that assists in the forward-direction movement of the suture thread fastening component 43 inside the housing 50, the suture thread fastening component 43 alone, even without the hollow component 42, can move in the forward direction. However, the timing of feed-out of the loop 82 and the timing of feed-out of the suture thread 1 must be adjusted in advance.

### Description of Reference Numerals

- 1: Suture thread
- 10: Operating part
- 20: Coupling part
- 30: Loop feeding/returning mechanism
- 31: Loop introduction plunger
- 31 a: Tip side coupling part
- 32: Hollow component for loop introduction
- 32a: Base side coupling part
- 40: Suture thread feeding mechanism
- 41: Suture thread introduction plunger
- 42: Hollow component
- 42a: Base end
- 43: Suture thread fastening component
- 50: Housing
- 51: Stopper
- 52: Supporting part
- 53: Supporting part
- 54: Inner wall surface
- 55: Guide hole
- 56: Flange part
- 60: Loop introduction needle
- 70: Suture thread introduction needle
- 80: Loop forming part
- 81: Rod-like shaft
- 82: Loop
- 83: Base side affixing part
- 100: Suturing implement
- 100A: Suturing implement
- 101: Abdominal wall
- 102: Visceral organ wall
- 103: Skin
- 400: Suture thread feeding mechanism
- 401: First guide
- 402: Second guide

## Claims

1. A medical suturing implement (100) comprising
a housing (50),
a loop introduction needle (60) provided on the tip side of said housing (50),
a suture thread introduction needle (70) in which suture thread (1) is housed, provided on the tip side of said housing (50) substantially parallel to and separated by a prescribed distance from said loop introduction needle (60),
a loop forming part (80) on the tip end of which a loop (82) is formed, which is moveably inserted inside said loop introduction needle (60),
a loop feeding/returning mechanism (30) which is adapted to move said loop forming part (80), provided inside said housing (50),
a suture thread feeding mechanism (40) which is adapted to feed out said suture thread (1), provided inside said housing (50),
**characterised by** an operating part (10) which is adapted to move said loop feeding/returning mechanism (30) and said suture thread feeding mechanism (40), provided inside said housing (50) so as to protrude from said housing (50), and
a coupling part (20) which is adapted to transfer the movement of said operating part (10) to said loop feeding/returning mechanism (30) and said suture thread feeding mechanism (40),
wherein, by said loop feeding/returning mechanism (30) and said suture thread feeding mechanism (40), the movement of said operating part (10) is transferred to said loop forming part (80) and said suture thread (1) with pre-adjusted timing between when said loop (82) is fed out from the tip end of said loop introduction needle (60) and when said suture thread (1) is fed out from the tip end of said suture thread introduction needle (70).

2. The medical suturing implement according to claim 1 wherein the movement of said operating part (10) is transferred with a time differential to said loop forming part (80) and said suture thread (1), and said suture thread (1) is fed out from the tip end of said suture thread introduction needle (70) after said loop (82) at least starts to be fed out from the tip end of said loop introduction needle (60).

3. The medical suturing implement (100) according to claim 1 or claim 2, wherein, by said loop feeding/returning mechanism (30) and said suture thread feeding mechanism (40), said loop (82) is housed inside said loop introduction needle (60) while the state where said suture thread (1) has been fed out from the tip end of said suture thread introduction needle (70) is maintained, and as a result, said suture thread (1) is grasped by the tip end of said loop introduction needle (60).

4. The medical suturing implement (100) according to claim 1, wherein said loop feeding/returning mechanism (30) comprises a hollow component for loop introduction (32) which feeds and returns said loop forming part (80), and a loop introduction plunger (31) configured such that the tip side can couple with the base side of said hollow component for loop introduction (32), and which transfers movement of said operating part (10) to said hollow component for loop introduction (32), and said suture thread feeding mechanism (40) comprises
a hollow component inside of which said suture thread is inserted (42), which is affixed to at least the base side of said housing (50), a suture thread introduction plunger (41) by which the movement of said operating part (10) is transferred, which has an inner cavity into which said hollow component (42) can be inserted, and a suture thread fastening component (43) which fastens said hollow component (42), said suture thread (1) or said suture thread introduction plunger (41) in accordance with the position to which it was moved on the tip side by said suture thread introduction plunger (41), and which is moved to the base side by said fastened suture thread introduction plunger (41), and by said loop feeding/returning mechanism (30), after said loop (82) has been fed out from the tip end of said loop introduction needle (60), the coupled state of said hollow component for loop introduction (32) and said loop introduction plunger (31) is released, and said loop introduction plunger (31) continues movement while maintaining the shape of said loop (82), and by said suture thread feeding mechanism (40), after the coupled state of said hollow component for loop introduction (32) and said loop introduction plunger (31) is released, said suture thread fastening component (43) which fastened said hollow component (42) comes out from said hollow component (42), and said suture thread (1) is fastened, and said suture thread (1) is fed out from the tip end of said suture thread introduction needle (70) such that it is inserted inside said loop (82) which has already been fed out or is starting to be fed out.

5. The medical suturing implement (100) according to claim 4, wherein, after said hollow component for loop introduction (32) runs into the tip side of said housing (50), the coupled state of said hollow component for loop introduction (32) and said loop introduction plunger (31) is released.

6. The medical suturing implement (100) according to claim 4, wherein the axial-direction length of said hollow component (42) is adjusted to a length such that said suture thread fastening component (43) comes out from said hollow component (42) after the coupled state of said hollow component for loop introduction (32) and said loop introduction plunger (31) is released.

7. The medical suturing implement (100) according to claim 4, wherein, after said suture thread fastening component (43) or a component that covers said suture thread fastening component (43) runs into the tip side of said housing (50), said suture thread introduction plunger (41) is inserted into said suture thread fastening component (43), and said suture thread fastening component (43) fastens said suture thread introduction plunger (41), and fastening to said suture thread (1) is released.

8. The medical suturing implement (100) according to claim 4, wherein, accompanying the movement of said operating part (10) toward the base side, said hollow component for loop introduction (32) and said loop introduction plunger (31) are again coupled, and said loop (82) is housed inside said loop introduction needle (60), and as a result, said suture thread (1) is grasped by the tip end of said loop introduction needle (60).

9. The medical suturing implement (100) according to claim 4, wherein a stopper (51) which restricts movement of said suture thread fastening component (43) toward the base side is provided on a part of the side surface of said housing (50), and said suture thread fastening component (43) that has run into said stopper (51) comes out from said suture thread introduction plunger (41) and said hollow component (42) is again fastened, and as a result, said suture thread (1) returns to the initial state while maintaining the unfastened state, and can be used continuously.

10. The medical suturing implement (100) according to claim 2, wherein an elastic component is provided in said housing (50), and said operating part (10) is moved utilizing the rebound force of said elastic component.

11. The medical suturing implement (100) according claim 3, wherein a lever is provided on a part of the side surface of said hollow component for loop introduction (32) such that it protrudes from said housing (50), and said hollow component for loop introduction (32) can be rotated via said lever.

12. The medical suturing implement (100) according to claim 1, wherein said loop feeding/returning mechanism (30) comprises a hollow component for loop introduction (32) which feeds and returns said loop forming part (80), and a loop introduction plunger (31) configured such that the tip side can couple with the base side of said hollow component for loop introduction (32), and which transfers movement of said operating part (10) to said hollow component for loop introduction (32), and said suture thread feeding mechanism (40) comprises a suture thread introduction plunger (41) by which the movement of said operating part (10) is transferred, and a suture thread fastening component (43) which fastens said suture thread (1) or said suture thread introduction plunger (41) in accordance with the position to which it was moved on the tip side by said suture thread introduction plunger (41), and which is moved to the base side by said fastened suture thread introduction plunger (41), and by said loop feeding/returning mechanism (30), after said loop (82) has been fed out from the tip end of said loop introduction needle (60), the coupled state of said hollow component for loop introduction (32) and said loop introduction plunger (31) is released, and said loop introduction plunger (31) continues movement while maintaining the shape of said loop (82), and by said suture thread feeding mechanism (40), said suture thread (1) which has been fastened by said suture thread fastening component (43) is fed out from the tip end of said suture thread introduction needle (70).

13. The medical suturing implement (100) according to claim 12, wherein, after said suture thread fastening component (43) runs into the tip side of said housing (50), said suture thread fastening component (43) fastens said suture thread introduction plunger (41), and fastening to said suture thread (1) is released.

## Patentansprüche

1. Medizinische Nahtvorrichtung (100), umfassend
ein Gehäuse (50),
eine Schlaufeneinführnadel (60), die an der Spitzenseite des Gehäuses (50) vorgesehen ist,
eine Nähfadeneinführnadel (70), in der ein Nähfaden (1) aufgenommen ist und die an der Spitzenseite des Gehäuses (50) im Wesentlichen parallel zu der Schlaufeneinführnadel (60) und um einen vorgegebenen Abstand davon entfernt vorgesehen ist,
einen Schlaufenbildungsteil (80), an dessen Spitzenende eine Schlaufe (82) gebildet wird, die beweglich in die Schlaufeneinführnadel (60) eingeführt wird,
einen Schlaufenzuführ-/-rückführmechanismus (30), der dazu angepasst ist, den Schlaufenbildungsteil (80) zu bewegen, und der im Gehäuse (50) vorgesehen ist,
einen Nähfadenzuführmechanismus (40), der dazu angepasst ist, den Nähfaden (1) abzugeben und der im Gehäuse (50) vorgesehen ist,
**gekennzeichnet durch** einen Betätigungsteil (10), der dazu angepasst ist, den Schlaufenzuführ-/-rückführmechanismus (30) und den Nähfadenzuführmechanismus (40) zu bewegen, und der derart im Gehäuse (50) vorgesehen ist, dass er aus dem Gehäuse (50) herausragt, und
einen Koppelungsteil (20), der dazu angepasst ist, die Bewegung des Betätigungsteils (10) auf den Schlaufenzuführ-/- rückführmechanismus (30) und den Nähfadenzuführmechanismus (40) zu übertragen,
wobei **durch** den Schlaufenzuführ-/-rückführmechanismus (30) und den Nähfadenzuführmechanismus (40) die Bewegung des Betätigungsteils (10) mit einer im Voraus festgelegten Zeit zwischen dem Zeitpunkt, an dem die Schlaufe (82) aus dem Spitzenende der Schlaufeneinführnadel (60) abgegeben wird, und dem Zeitpunkt, an dem der Nähfaden (1) aus dem Spitzenende der Nähfadeneinführnadel (70) abgegeben wird, auf den Schlaufenbildungsteil (80) und den Nähfaden übertragen wird.

2. Medizinische Nahtvorrichtung nach Anspruch 1, wobei die Bewegung des Betätigungsteils (10) mit einer Zeitdifferenz zu dem Schlaufenbildungsteil (80) und dem Nähfaden (1) übertragen wird, und der Nähfaden (1) aus dem Spitzenende der Nähfadeneinführnadel (70) abgegeben wird, nachdem die Schlaufe (82) wenigstens begonnen hat, aus dem Spitzenende der Schlaufeneinführnadel (60) abgegeben zu werden.

3. Medizinische Nahtvorrichtung (100) nach Anspruch 1 oder Anspruch 2, wobei, durch den Schlaufenzuführ-/- rückführmechanismus (30) und den Nähfadenzuführmechanismus (40), die Schlaufe (82) im Inneren der Schlaufeneinführnadel (60) aufgenommen ist, während der Zustand, in dem der Nähfaden (1) aus dem Spitzenende der Nähfadeneinführnadel (70) abgegeben wurde, aufrechterhalten wird, wodurch der Nähfaden (1) vom Spitzenende der Schlaufeneinführnadel (60) ergriffen wird.

4. Medizinische Nahtvorrichtung (100) nach Anspruch 1, wobei der Schlaufenzuführ-/-rückführmechanismus (30) eine Hohlkomponente zur Schlaufeneinführung (32), die den Schlaufenbildungsteil (80) zuführt und zurückführt, und einen Schlaufeneinführkolben (31) umfasst, der derart konfiguriert ist, dass die Spitzenseite an die Basisseite der Hohlkomponente zur Schlaufeneinführung (32) ankoppeln kann, und der die Bewegung des Betätigungsteils (10) auf die Hohlkomponente zur Schlaufeneinführung (32) überträgt, und wobei der Nähfadenzuführmechanismus (40) Folgendes umfasst eine Hohlkomponente, in deren Innerem der Nähfaden eingeführt ist (42), die wenigstens an der Basisseite des Gehäuses (50) befestigt ist, einen Nähfadeneinführkolben (41), durch den die Bewegung des Betätigungsteils (10) übertragen wird und der einen Innenhohlraum aufweist, in den die Hohlkomponente (42) eingeführt werden kann, und eine Nähfadenbefestigungskomponente (43), die die Hohlkomponente (42), den Nähfaden (1) oder den Nähfadeneinführkolben (41) entsprechend der Position befestigt, an die sie durch den Nähfadeneinführkolben (41) zur Spitzenseite hin bewegt wurde, und die durch den befestigten Nähfadeneinführkolben (41) zur Basisseite bewegt wird, und durch den Schlaufenzuführ-/-rückführmechanismus(30), nachdem die Schlaufe (82) aus dem Spitzenende der Schlaufeneinführnadel (60) abgegeben wurde, der gekoppelte Zustand der Hohlkomponente zur Schlaufeneinführung (32) und des Schlaufeneinführkolbens (31) freigegeben wurde und der Schlaufeneinführkolben (31) die Bewegung fortsetzt, während die Form der Schlaufe (82) beibehalten wird, und durch den Nähfadenzuführmechanismus (40), nachdem der gekoppelte Zustand der Hohlkomponente zur Schlaufeneinführung (32) und des Schlaufeneinführkolbens (31) freigegeben wurde, wobei die Nähfadenbefestigungskomponente (43), die die Hohlkomponente (42) befestigt hat, aus der Hohlkomponente (42) tritt und der Nähfaden (1) befestigt wird, und der Nähfaden (1) aus dem Spitzenende der Nähfadeneinführnadel (70) abgegeben wird, derart, dass er in die Schlaufe (82) eingeführt wird, die bereits abgegeben wurde oder begonnen hat, abgegeben zu werden.

5. Medizinische Nahtvorrichtung (100) nach Anspruch 4, wobei, nachdem die Hohlkomponente zur Schlaufeneinführung (32) in die Spitzenseite des Gehäuses (50) gelangt ist, der gekoppelte Zustand der Hohlkomponente zur Schlaufeneinführung (32) und des Schlaufeneinführkolbens (31) freigegeben wird.

6. Medizinische Nahtvorrichtung (100) nach Anspruch 4, wobei die Länge der Hohlkomponente (42) in Axialrichtung auf eine Länge angepasst ist, derart, dass die Nähfadenbefestigungskomponente (43) aus der Hohlkomponente (42) tritt, nachdem der gekoppelte Zustand der Hohlkomponente zur Schlaufeneinführung (32) und des Schlaufeneinführkolbens (31) freigegeben wurde.

7. Medizinische Nahtvorrichtung (100) nach Anspruch 4, wobei, nachdem die Nähfadenbefestigungskomponente (43) oder eine Komponente, die die Nähfadenbefestigungskomponente (43) abdeckt, in die Spitzenseite des Gehäuses (50) gelangt ist, der Nähfadeneinführkolben (41) in die Nähfadenbefestigungskomponente (43) eingeführt wird und die Nähfadenbefestigungskomponente (43) den Nähfadeneinführkolben (41) befestigt und die Befestigung am Nähfaden (1) freigegeben wird.

8. Medizinische Nahtvorrichtung (100) nach Anspruch 4, wobei die Hohlkomponente zur Schlaufeneinführung (32) und der Schlaufeneinführkolben (31) in Begleitung der Bewegung des Betätigungsteils (10) zur Basisseite hin erneut gekoppelt werden und die Schlaufe (82) in der Schlaufeneinführnadel (60) aufgenommen wird, wodurch der Nähfaden (1) vom Spitzenende der Schlaufeneinführnadel (60) ergriffen wird.

9. Medizinische Nahtvorrichtung (100) nach Anspruch 4, wobei ein Anschlag (51), der die Bewegung der Nähfadenbefestigungskomponente (43) zur Basisseite hin einschränkt, an einem Teil der Seitenfläche des Gehäuses (50) vorgesehen ist, und die Nähfadenbefestigungskomponente (43), die sich gegen den Anschlag (51) bewegt hat, aus dem Nähfadeneinführkolben (41) austritt und die Hohlkomponente (42) erneut befestigt wird, und wodurch der Nähfaden (1) in den Ausgangszustand zurückkehrt, während der nicht befestigte Zustand beibehalten wird, und kontinuierlich benutzt werden kann.

10. Medizinische Nahtvorrichtung (100) nach Anspruch 2, wobei eine elastische Komponente in dem Gehäuse (50) vorgesehen ist und der Betätigungsteil (10) unter Ausnutzung der Rückstellkraft der elastischen Komponente bewegt wird.

11. Medizinische Nahtvorrichtung (100) nach Anspruch 3, wobei ein Hebel an einem Teil der Seitenfläche der Hohlkomponente zur Schlaufeneinführung (32) vorgesehen ist, derart, dass er aus dem Gehäuse (50) vorspringt, und die Hohlkomponente zur Schlaufeneinführung (32) über den Hebel gedreht werden kann.

12. Medizinische Nahtvorrichtung (100) nach Anspruch 1, wobei der Schlaufenzuführ-/-rückführmechanismus (30) eine Hohlkomponente zur Schlaufeneinführung (32), die den Schlaufenbildungsteil (80) zuführt und zurückführt, und einen Schlaufeneinführkolben (31) umfasst, der derart konfiguriert ist, dass die Spitzenseite an die Basisseite der Hohlkomponente zur Schlaufeneinführung (32) ankoppeln kann, und der die Bewegung des Betätigungsteils (10) auf die Hohlkomponente zur Schlaufeneinführung (32) überträgt, und der Nähfadenzuführmechanismus (40) einen Nähfadeneinführkolben (41), durch den die Bewegung des Betätigungsteils (10) übertragen wird, und eine Nähfadenbefestigungskomponente (43) umfasst, die den Nähfaden (1) oder den Nähfadeneinführkolben (41) entsprechend der Position befestigt, in die sie an der Spitzenseite durch den Nähfadeneinführkolben (41) bewegt wurde, und die durch den befestigten Nähfadeneinführkolben (41) auf die Basisseite bewegt wird, und durch den Schlaufenzuführ-/- rückführmechanismus (30), nachdem die Schlaufe (82) aus dem Spitzenende der Schlaufeneinführnadel (60) abgegeben wurde, der gekoppelte Zustand der Hohlkomponente zur Schlaufeneinführung (32) und des Schlaufeneinführkolbens (31) freigegeben wird und der Schlaufeneinführkolben (31) die Bewegung fortsetzt, während die Form der Schlaufe (82) beibehalten wird, und durch den Nähfadenzuführmechanismus (40), wobei der Nähfaden (1), der von der Nähfadenbefestigungskomponente (43) befestigt wurde, aus dem Spitzenende der Nähfadeneinführnadel (70) abgegeben wird.

13. Medizinische Nahtvorrichtung (100) nach Anspruch 12, wobei, nachdem die Nähfadenbefestigungskomponente (43) in die Spitzenseite des Gehäuses (50) gelangt ist, die Nähfadenbefestigungskomponente (43) den Nähfadeneinführkolben (41) befestigt und die Befestigung am Nähfaden (1) freigegeben wird.

## Revendications

1. Instrument de suture médicale (100) comprenant
un logement (50),
une aiguille d'introduction de boucle (60) prévue sur le côté pointe dudit logement (50),
une aiguille d'introduction de fil de suture (70) dans laquelle du fil de suture (1) est logé, prévue sur le côté pointe dudit logement (50) sensiblement parallèle à et séparée d'une distance prescrite de ladite aiguille d'introduction de boucle (60),
une pièce formant une boucle (80) sur l'extrémité de pointe de laquelle une boucle (82) est formée, qui est insérée de manière déplaçable à l'intérieur de ladite aiguille d'introduction de boucle (60),
un mécanisme d'amenée/de retour de boucle (30) qui est adapté pour déplacer ladite pièce formant une boucle (80), prévu à l'intérieur dudit logement (50),
un mécanisme d'amenée de fil de suture (40) qui est adapté pour amener ledit fil de suture (1), prévu à l'intérieur dudit logement (50),
**caractérisé par**
une pièce fonctionnelle (10) qui est adaptée pour déplacer ledit mécanisme d'amenée/de retour de boucle (30) et ledit mécanisme d' amenée de fil de suture (40), prévue à l'intérieur dudit logement (50) de manière à dépasser dudit logement (50), et
une pièce de couplage (20) qui est adaptée pour transférer le mouvement de ladite pièce fonctionnelle (10) audit mécanisme d'amenée/de retour de boucle (30) et audit mécanisme d'amenée de fil de suture (40),
dans lequel, par ledit mécanisme d'amenée/de retour de boucle (30) et ledit mécanisme d' amenée de fil de suture (40), le mouvement de ladite pièce fonctionnelle (10) est transféré à ladite pièce formant une boucle (80) et audit fil de suture (1) avec synchronisation préréglée entre le moment où ladite boucle (82) est amenée hors de l'extrémité de pointe de ladite aiguille d'introduction de boucle (60) et le moment où ledit fil de suture (1) est amené hors de l'extrémité de pointe de ladite aiguille d'introduction de fil de suture (70).

2. Instrument de suture médicale selon la revendication 1, dans lequel le mouvement de ladite pièce fonctionnelle (10) est transféré avec un différentiel de temps à ladite pièce formant une boucle (80) et audit fil de suture (1), et ledit fil de suture (1) est amené hors de l'extrémité de pointe de ladite aiguille d'introduction de fil de suture (70) après que ladite boucle (82) a au moins commencé à être amenée hors de l'extrémité de pointe de ladite aiguille d'introduction de boucle (60).

3. Instrument de suture médicale (100) selon la revendication 1 ou 2, dans lequel, par ledit mécanisme d'amenée/de retour de boucle (30) et ledit mécanisme d'amenée de fil de suture (40), ladite boucle (82) est logée à l'intérieur de ladite aiguille d'introduction de boucle (60) tant qu'est maintenu l'état où ledit fil de suture (1) a été amené hors de l'extrémité de pointe de ladite aiguille d'introduction de fil de suture (70), et de ce fait, ledit fil de suture (1) est saisi par l'extrémité de pointe de ladite aiguille d'introduction de boucle (60).

4. Instrument de suture médicale (100) selon la revendication 1, dans lequel ledit mécanisme d'amenée/de retour de boucle (30) comprend un composant creux pour l'introduction d'une boucle (32) qui amène et retourne ladite pièce formant une boucle (80), et un piston d'introduction de boucle (31) configuré de sorte que le côté pointe peut se coupler au côté base dudit composant creux pour l'introduction d'une boucle (32), et qui transfère le mouvement de ladite pièce fonctionnelle (10) audit composant creux pour l'introduction d'une boucle (32), et ledit mécanisme d'amenée de fil de suture (40) comprend
un composant creux à l'intérieur duquel ledit fil de suture est inséré (42), qui est attaché au moins au côté base dudit logement (50), un piston d'introduction de fil de suture (41) par lequel le mouvement de ladite pièce fonctionnelle (10) est transféré, qui a une cavité intérieure dans laquelle peut être inséré ledit composant creux (42), et un composant de fixation du fil de suture (43) qui fixe ledit composant creux (42), ledit fil de suture (1) ou ledit piston d'introduction de fil de suture (41) en fonction de la position jusqu'à laquelle il a été déplacé sur le côté pointe par ledit piston d'introduction de fil de suture (41), et qui est déplacé jusqu'au côté base par ledit piston d'introduction de fil de suture fixé (41), et par ledit mécanisme d'amenée/de retour de boucle (30), après que ladite boucle (82) a été amenée hors de l'extrémité de pointe de ladite aiguille d'introduction de boucle (60), l'état couplé dudit composant creux pour l'introduction d'une boucle (32) et dudit piston d'introduction de boucle (31) est libéré, et ledit piston d'introduction de boucle (31) continue son déplacement tout en maintenant la forme de ladite boucle (82), et par ledit mécanisme d'amenée de fil de suture (40), après libération de l'état couplé dudit composant creux pour l'introduction d'une boucle (32) et dudit piston d'introduction de boucle (31), ledit composant de fixation du fil de suture (43) qui a fixé ledit composant creux (42) sort dudit composant creux (42), et ledit fil de suture (1) est fixé, et ledit fil de suture (1) est amené hors de l'extrémité de pointe de ladite aiguille d'introduction de fil de suture (70) de sorte qu'il est inséré à l'intérieur de ladite boucle (82) qui a déjà été amenée ou commence à être amenée.

5. Instrument de suture médicale (100) selon la revendication 4, dans lequel, après que ledit composant creux pour l'introduction d'une boucle (32) a buté contre le côté pointe dudit logement (50), l'état couplé dudit composant creux pour l'introduction d'une boucle (32) et dudit piston d'introduction de boucle (31) est libéré.

6. Instrument de suture médicale (100) selon la revendication 4, dans lequel la longueur en direction axiale dudit composant creux (42) est ajustée à une longueur telle que ledit composant de fixation du fil de suture (43) sort dudit composant creux (42) après libération de l'état couplé dudit composant creux pour l'introduction d'une boucle (32) et dudit piston d'introduction de boucle (31).

7. Instrument de suture médicale (100) selon la revendication 4, dans lequel, après que ledit composant de fixation du fil de suture (43) ou un composant qui recouvre ledit composant de fixation du fil de suture (43) a buté contre le côté pointe dudit logement (50), ledit piston d'introduction de fil de suture (41) est inséré dans ledit composant de fixation du fil de suture (43), et ledit composant de fixation du fil de suture (43) fixe ledit piston d'introduction de fil de suture (41), et la fixation audit fil de suture (1) est libérée.

8. Instrument de suture médicale (100) selon la revendication 4, dans lequel, en accompagnant le mouvement de ladite pièce fonctionnelle (10) vers le côté base, ledit composant creux pour l'introduction d'une boucle (32) et ledit piston d'introduction de boucle (31) sont à nouveau couplés, et ladite boucle (82) est logée à l'intérieur de ladite aiguille d'introduction de boucle (60), et de ce fait, ledit fil de suture (1) est saisi par l'extrémité de pointe de ladite aiguille d'introduction de boucle (60).

9. Instrument de suture médicale (100) selon la revendication 4, dans lequel un élément de butée (51) qui limite le mouvement dudit composant de fixation du fil de suture (43) en direction du côté base est prévu sur une partie de la surface latérale dudit logement (50), et ledit composant de fixation du fil de suture (43) qui a buté contre ledit élément de butée (51) sort dudit piston d'introduction de fil de suture (41) et ledit composant creux (42) est à nouveau fixé, et de ce fait, ledit fil de suture (1) retourne à l'état initial tout en maintenant l'état non fixé, et peut être utilisé en continu.

10. Instrument de suture médicale (100) selon la revendication 2, dans lequel un composant élastique est prévu dans ledit logement (50), et ladite pièce fonctionnelle (10) est déplacée en utilisant la force de rebond dudit composant élastique.

11. Instrument de suture médicale (100) selon la revendication 3, dans lequel un levier est prévu sur une partie de la surface latérale dudit composant creux pour l'introduction d'une boucle (32) de telle sorte qu'il dépasse dudit logement (50), et ledit composant creux pour l'introduction d'une boucle (32) peut être pivoté via ledit levier.

12. Instrument de suture médicale (100) selon la revendication 1, dans lequel ledit mécanisme d'amenée/de retour de boucle (30) comprend un composant creux pour l'introduction d'une boucle (32) qui amène et retourne ladite pièce formant une boucle (80), et un piston d'introduction de boucle (31) configuré de sorte que le côté pointe peut se coupler avec le côté base dudit composant creux pour l'introduction d'une boucle (32), et qui transfère le mouvement de ladite pièce fonctionnelle (10) audit composant creux pour l'introduction d'une boucle (32), et ledit mécanisme d'amenée de fil de suture (40) comprend un piston d'introduction de fil de suture (41) par lequel le mouvement de ladite pièce fonctionnelle (10) est transféré, et un composant de fixation du fil de suture (43) qui fixe ledit fil de suture (1) ou ledit piston d'introduction de fil de suture (41) en fonction de la position jusqu'à laquelle il a été déplacé sur le côté pointe par ledit piston d'introduction de fil de suture (41), et qui est déplacé jusqu'au côté base par ledit piston d'introduction de fil de suture (41) fixé, et par ledit mécanisme d'amenée/de retour de boucle (30), après que ladite boucle (82) a été amenée hors de l'extrémité de pointe de ladite aiguille d'introduction de boucle (60), l'état couplé dudit composant creux pour l'introduction d'une boucle (32) et dudit piston d'introduction de boucle (31) est libéré, et ledit piston d'introduction de boucle (31) continue son déplacement tout en maintenant la forme de ladite boucle (82), et par ledit mécanisme d'amenée de fil de suture (40), ledit fil de suture (1) qui a été fixé par ledit composant de fixation du fil de suture (43) est amené hors de l'extrémité de pointe de ladite aiguille d'introduction de fil de suture (70).

13. Instrument de suture médicale (100) selon la revendication 12, dans lequel, après que ledit composant de fixation du fil de suture (43) a buté contre le côté pointe dudit logement (50), ledit composant de fixation du fil de suture (43) fixe ledit piston d'introduction de fil de suture (41), et la fixation audit fil de suture (1) est libérée.
